# EUROPEAN PATENT APPLICATION

(11) **EP 2 186 505 A1**
(43) Date of publication of application: **19.05.2010**
(21) Application number: 09175811.0
(22) Date of filing: 12.11.2009
(51) Int. Cl.: A61K 8/24, A61K 8/36, A61Q 11/02

(54) **Liquid cleaning agent for removable dental prostheses and equivalent devices**

(30) Priority: 12.11.2008 FI 20086074
(71) Applicant: Hammaslaboratorio Hyvä Hammas Oy, 96100 Rovaniemi (FI)
(72) Inventor: Isojärvi, Jyrki, 96100, Rovaniemi (FI)
(74) Representative: Laurinolli, Tapio Kullervo

(57) **Abstract**

Liquid cleaning agent for dental prostheses and equivalent devices is **characterized in that** it is an aqueous solution the pH of which is less than 2 and which includes ortho-phosphorus acid and acetic acid in the ratio of from 10:1 to 5:1 and suitable bases to produce a buffer effect against the adverse effects of the acids on the materials of the devices.

## Description

### FIELD OF THE INVENTION

The invention is related to cleaning of removable dental prostheses and equivalent devices and especially to a liquid cleaning agent for that purpose.

### BACKGROUND OF THE INVENTION

At present, soluble cleaning agents in tablet form are generally used for cleaning of removable dental prostheses and equivalent devices, e.g. orthodontic and occlusal appliances. It is clear that the solid state sets limits to that which substances may be used in a cleaning agent. Therefore, there is room for improvements in the efficiency of cleaning agents in tablet form. In the use of them, also dosing and adjustment of the strength of the working solution are to some extent problematic.

A background for this invention is a need to achieve a liquid, easy to use and effective cleaning agent.

Firstly, a requirement for a good cleaning agent is that it kills effectively the bacteria and removes plaque built up on the surfaces. Additionally, it should also loosen calculus accumulated in the long term on the structures and remove stains. A prosthesis or equivalent device cleaned by the agent should also have a fresh odor and taste after a treatment.

A further requirement for a cleaning agent is that it must not even as a strong solution damage materials of the dental prostheses and equivalent devices, which materials include plastic materials based on polymethylmetacrylate, vinyl acetate and ethyl vinyl acetate and silicone based hard and soft plastic plate materials, ceramic materials like zirconium and aluminium oxide based materials, and metal materials like chromium-cobalt, chromium-nickel, titanium, gold, platinum and palladium based dental metal alloys.

### SUMMARY OF THE INVENTION

An object of the invention is to present a cleaning agent for removable dental prostheses and equivalent devices fulfilling the above needs and requirements.

To achieve this object, a liquid cleaning agent for dental prostheses and equivalent devices according to the invention is **characterized in that** it is an aqueous solution the pH of which is less than 2 and which includes ortho-phosphorus acid and acetic acid in the ratio of from 10:1 to 5:1 and suitable bases to produce a buffer effect against the adverse effects of the acids on the materials of the devices.

The cleaning agent includes ortho-phosphorus acid and acetic acid advantageously in the ratio of from 8:1 1 to 6:1.

The pH of the cleaning agent is advantageously within the range of 1.4 to 1.7, and the preferred pH value is 1.5 to 1.6.

The content of ortho-phosphorus acid is advantageously 1.7 to 1.8 weight percent and the content of acetic acid is 0.2 to 0.3 weight percent.

The cleaning agent may include at least one of the following bases: sodium hydroxide, calcium carbonate and potassium carbonate. The content of sodium hydroxide may be e.g. 0.002 to 0.003, the content of calcium carbonate e.g. 0.007 to 0.008, and the content of potassium carbonate e.g. 0.0004 to 0.0005 weight percent.

### DETAILED DESCRIPTION OF THE INVENTION

When developing the cleaning agent according to the invention, a starting point was that it is an aqueous solution to be diluted with water and that it is acid enough so that, when diluted in home life according to instructions, the acidity of the cleaning solution is high enough in view of killing the bacteria. From this starting point, the conclusion was that the pH value must be less than 2 and advantageously within the range of 1.4 to 1.7. The instruction in the production of the solution is that the pH is within 1.5 to 1.6.

When selecting active ingredients, we resulted in the combination of ortho-phosphorus acid H₃PO₄ and acetic acid CH₃COOH. From these acids, the primary task of the ortho-phosphorus acid is to remove calculus and the task of the acetic acid, which is an organic acid, is to remove plaque and bacteria as well as stains.

The experiments indicated that the suitable ratio of the ortho-phosphorus acid and acetic acid in the cleaning solution is within the range of 10:1 to 5:1 and preferably within the range of 8:1 1 to 6:1. Defining the contents, a useful instruction is that the content of the ortho-phosphorus acid in the solution is 1.7 to 1.8 weight percent and the content of the acetic acid is 0.2 to 0.3 weight percent.

To avoid possible adverse effects of the acids on the materials of a dental prosthesis or other equivalent device, the solution must preferably include suitable bases for buffering against these effects. Such bases include e.g. sodium hydroxide NaOH, calcium carbonate CaCO₃ and potassium carbonate K₂CO₃. Defining the contents, a useful instruction is that the cleaning solution includes these ingredients as follows: sodium hydroxide 0.002 to 0.003, calcium carbonate 0.007 to 0.008, and potassium carbonate 0.0004 to 0.0005 weight percent.

Instead of the above bases and salts some other ones may be used which have equivalent effects. It is probable that instead of sodium hydroxide potassium hydroxide KOH, instead of calcium carbonate baking soda i.e. sodium hydrogen carbonate NaHCO₃, and instead of potassium carbonate possibly soda i.e. sodium bicarbonate Na₂CO₃ could be used. E.g. from the above mentioned bases and salts it is possible on the basis of professional expertise and experiments to compose many combinations of bases and salts with various contents which realize the desired buffer effect and are otherwise suitable for the purpose.

A fresh taste for the cleaning agent is obtained e.g. by using suitable flavouring preparation like mint preparation. As to the colour of the cleaning agent, it may be improved by means of suitable food colours.

For improving the preservability of the cleaning solution, it is advantageously made by using hundred per cent ion-exchanged water.

The solution has been tested by mixing working solutions of various strengthes with normal tap water. The contents of the cleaning agent in the cleaning solutions used in the tests have been 100, 50, 25, 15, 10 and 5 percent. An object to be cleaned has been kept in the solution at 20 degrees centigrade for the following periods: 15 minutes, 30 minutes, 1 hour, 2 hours, 4 hours, 8 hours, 12 hours, and 24 hours. Furthermore, intensification of the cleaning has been cleared up by placing the object to be cleaned in the cleaning solution in an ultrasonic cleaning device for 30 minutes.

According to the tests, for regular cleaning in home conditions a working solution with the cleaning agent content of 10 to 15 percent and a keeping time of 20 to 60 minutes in the solution are enough. For removing a very big old mass of calculus, a cleaning agent content of 50 percent and keeping time of even 12 hours in the solution may be necessary. The use of an ultrasonic cleaning device makes cleaning faster and more effective wherein the use of such device is recommendable at least in professional use. Not even the use of the cleaning agent without any dilution for 24 hours did cause any damages to the tested structures.

The invention may vary within the scope of the accompanying claims.

## Claims

1. Liquid cleaning agent for dental prostheses and equivalent devices, **characterized in that** it is an aqueous solution the pH of which is less than 2 and which includes ortho-phosphorus acid and acetic acid in the ratio of from 10:1 1 to 5:1 1 and suitable bases to produce a buffer effect against the adverse effects of the acids on the materials of the devices.

2. Liquid cleaning agent according to claim 1, **characterized in that** it includes ortho-phosphorus acid and acetic acid in the ratio of from 8:1 1 to 6:1.

3. Liquid cleaning agent according to claim 1, **characterized in that** its pH value is 1.4 to 1.7.

4. Liquid cleaning agent according to claim 1, **characterized in that** its pH value is 1.5 to 1.6.

5. Liquid cleaning agent according to claim 1, **characterized in that** the content of ortho-phosphorus acid is 1.7 to 1.8 weight percent.

6. Liquid cleaning agent according to claim 1, **characterized in that** the content of acetic acid is 0.2 to 0.3 weight percent.

7. Liquid cleaning agent according to claim 1, **characterized in that** it includes at least one of the following bases: sodium hydroxide, calcium carbonate and potassium carbonate.

8. Liquid cleaning agent according to claim 7, **characterized in that** the content of sodium hydroxide is 0.002 to 0.003 weight percent.

9. Liquid cleaning agent according to claim 7, **characterized in that** the content of calcium carbonate is 0.007 to 0.008 weight percent.

10. Liquid cleaning agent according to claim 7, **characterized in that** the content of potassium carbonate is 0.0004 to 0.0005 weight percent.
